# EUROPEAN PATENT APPLICATION

(11) **EP 3 320 898 A1**
(43) Date of publication of application: **16.05.2018**
(21) Application number: 16198482.8
(22) Date of filing: 11.11.2016
(51) Int. Cl.: A61K 9/70, B33Y 10/00, B33Y 30/00

(54) **DOSAGE FORM COMPRISING A CARRIER STRUCTURE AND METHOD FOR MANUFACTURING THE DOSAGE FORM**

(71) Applicant: ratiopharm GmbH, 89079 Ulm (DE)
(72) Inventor: Rommel, Steve, 71287 Weissach (DE)
(74) Representative: Rösler, Uwe

(57) **Abstract**

The invention refers to a dosage form comprising a carrier with at least two sections adjoining each other integrally by a linearly solid joint, at least one sections provides at least one active pharmaceutical ingredient (API) and/or pharmaceutically acceptable excipient and/or drug product formulation. Further the invention concerns to a method for manufacturing the dosage form of the before kind.

The inventive idea is characterized by manufacturing at least the solid joint by way of a generative manufacturing method. This technique enables to design the solid joint with a maximum degree of freedom in view of shape, size, material to name a few and to provide basis for functionality.

## Description

### Technical Field

The invention refers to a dosage form comprising a carrier structure with at least two sections adjoining each other integrally by a linearly solid joint, at least one section provides at least one active pharmaceutical ingredient (API) and/or pharmaceutically acceptable excipient and/or drug product formulation. Further the invention concerns to a method for manufacturing the dosage form of the before kind.

### Background of the Invention

One aspect of designing new dosage forms is to release active pharmaceutical ingredients (API), pharmaceutically acceptable excipient and drug product formulation only at intracorporal regions of therapeutically interests to avoid or at least to minimize side effects of each active pharmaceutical ingredient released into the body.

For ease of further description, the abbreviation "API" is intended to mean at all one of the following substances: Active pharmaceutical ingredient, pharmaceutically acceptable excipient and drug product formulation.

One way of controlling the intracorporal drug delivery is to use encapsulated dosage forms providing a predetermined dissolution behavior in intracorporal liquid environment. Hereto typical dosage forms have a tablet coat and a tablet core which tablet coat has a temporal resolution in the body depending on the intracorporal residence time and intracorporal chemical and biological liquid milieu, so that the drug delivery can be effected with a high spatial accuracy.

Further so called multi-layered degradable drug formulations are known for the controlled release of API such that an immediate release shortly after intake of the dosage form and/or sustained release of active agents are possible.

Typically the tablet core contains the at least one API. The tablet core can be in pure form of API or API is an additional compound in a solid, liquid, granulated or powdery material matrix.

A new approach for delivering APIs using dosage forms like a pill or tablet concerns a special kind of configuration of the tablet or the tablet core which is in form of an unfoldable structure like a foldable surface element or body, which can be transformed by pure folding from an unfolded state into a small and compact shape in which parts of the structure are close together. In the folded state the structure is housed in a tablet or pill capsule or outer coating which encloses the foldable inner structure hermetically.

The foldable structure can be of pure API being of solid nature or provides a carrier surface element or body for example in form of a film or sheet onto which at least one API is applied at least in regions.

Once the dosage form is consumed and the capsule or outer coating serving mainly as a fixation of the folded inner structure is dissolved the folded structure starts to self-unfold by changing its shape, forming a large freely accessible surface which allows a higher amount of release of API over the increased surface barrier which unfolds intracorporaly for example in the digestive systems.

Depending on size and shape of the unfolded structure another aspect becomes important which concerns the retention time in a specific portion of a hollow organ. If the unfolded structure is sufficiently large and/or provides a sufficiently complex spatial form than the unfolded structure contact an inner wall of a hollow organ and will get caught there at least for a while during which the at least one API will be released. The unfolded structure will stay at a targeted intracorporal area until the structure is fully dissolved and broken down into smaller portions which can be excreted naturally.

In connection with the foregoing dosage the document WO 2009/144558 discloses a degradable, multi-layered gastroretentiv drug formulation for the sustained release of an active agent in the stomach providing an internal layer comprising at least an active agent and a degradable polymer which is covered at both sides with a hydrophilic polymer membrane. The membrane has predetermined length greater than 20 mm in a planar orientation and the membrane and internal layer being arranged in an accordion folded orientation sufficiently compact to be placed within a capsule dissolvable within the stomach. The membrane and internal layer developing sufficient mechanical force to unfold from the initial accordion folded orientation to a length of at least 20mm within 30 minutes of being exposed to gastric medium. The membrane permitting passage of gastric media from the environment to the internal layer and permitting passage of the active agent from the internal layer through the membrane to the environment.

Based on the same idea the document CA 2815959 A1 discloses a so called accordion pill comprising levodopa for an improved treatment of Parkinson's decease symptoms. A comparable accordion pill is disclosed also in US 2014/0017303 A1.

Finally the document WO 2015/191920 A1 discloses a dosage form providing a star or ring shaped unfolded structure which is placed in compact folded shape in a capsule.

### Summary of the Invention

It is a general object of the present invention to provide a dosage form comprising a carrier with at least two sections joining each other integrally by a linearly solid joint at least one section provides at least one API which permits an intracorporal drug delivery that is individually adapted to a person. It is further an object of the invention to create the possibility to take effect on the drug delivery by the dosage form from extracorporal position. Finally it is an object of the invention to ensure that the ingested dosage form can be completely dissolved or removed extracorporally safely.

The object is achieved by a dosage form given in claims 1 and 2. Subject matter of claims 14 and 15 relates to a method for manufacturing the dosage form according to claim 1. Claim 16 specifies a method for manufacturing the dosage form according to claim 2. All features of the independent claims can be modified advantageously by the features disclosed in the corresponding subclaims as well in the following description especially referring to preferred embodiments.

The inventive idea concerns the way of manufacturing at least the solid joint between the two sections joining each other integrally which is produced by a generative manufacturing method, preferably by way of an additive manufacturing process.

The dosage form uses the before mentioned mechanism of a foldable and unfoldable carrier structure which can be of two or three dimensional shape and form. Said carrier structure is of a solid material onto or into which the at least one API is attached by way of coating, spraying, mixing or all other known techniques of material adding. It is also conceivable that the carrier structure consists of API only.

The main advantage of using a generative manufacturing method for manufacturing at least the solid joint is to reach a maximum degree of freedom in view of shape, size, choice of material for designing and realizing the solid joint into which smart functionality can be implemented respectively can be integrated.

Preferably the solid joint differs from adjoining sections of the carrier structure at least in one of the following features: Material, density, flexibility, geometry concerning thickness and/or shape, solubility in liquid, and thermal, electrical, magnetical, ultrasound and/or electromagnetic absorption ability.

The choice of material which can be processed generatively depends on the type of process which is used for manufacturing the solid joint. Basically the term "generative manufacturing method" is a preamble covering all additive fabrication or manufacturing methods which are carried out directly on a binary data set which describes all relevant parameters of a 3D-model or structure. All different generative methods starting the process with formless material like liquids, powders and so on or form neutral materials like band-or wire like material and transform the starting material by means of chemical and/or physical processes into a stiffened structure being installed layer by layer.

One of the most suitable generative manufacturing method concerns the 3D printing technic or inkjet technology in which a powder bed is prepared in layers into which a binder liquid is deposited on basis of a defined geometrically trajectory clearly. Beside of the before mentioned powder bed based 3D-printing alternative powder bed based generative manufacturing methods are suitable like electron-beam melting, selective laser melting, selective heat sintering, selective laser sintering, direct metal laser sintering. In further preferred embodiments it is also conceivable to use extrusion based generative manufacturing method like fused deposition modelling or fused filament fabrication.

To complete suitable generative manufacturing techniques it is also conceivable to use stereo lithography or digital light processing technique to realize the at least one solid joint. Depending on the material to be used for manufacturing the solid joint one of the before mentioned generative manufacturing method can be selected freely.

Alternatively of using generative manufacturing technique of building the solid joint it is proposed inventively also to produce the solid joint in way of laser beam writing, i.e. the at least one surface of the carrier structure is locally irradiated by a laser beam which is focused onto the surface for transforming and/or removing material of the carrier structure partially. The laser beam is deflected such, that the beam follows a straight or curved line pattern on the surface of the carrier structure preferably. The interaction of the laser beam and the material of the carrier structure may softened or introduce flexibility into the material of the carrier structure locally so that the at least two sections of the carrier structure adjoining each other integrally by the linearly solid joint can swing once or multiple times in a predetermined manner around the solid joint.

Alternatively or in combination with using the before described laser beam writing technique the carrier structure can be mechanically weakened in a controlled manner along a predetermined linear or curved pattern on the surface of the carrier structure by introducing at least one surface cut at least at one surface of the carrier structure by a mechanical cutting tool which penetrates the surface of the carrier structure without complete separation. The movement of the cutting tool is performed on basis of the binary data set. It is conceivable that the technique of laser beam writing and mechanical cutting can be performed in combination, for example in a first step the cutting technique may be applied followed by laser beam writing along the cutting pattern or vice versa.

In a preferred embodiment of the invention the solid joint between two adjoining sections of a foldable structure is made of an elastic material which ensures a self-expanding unfolding and to remain in the unfolded state when the tablet coat is dissolved. Not necessarily a tablet coat like a capsule, in which the folded carrier structure is placed, is necessary to keep the carrier structure in the folded sate. In an alternative solution the carrier structure provides a holding mechanism connected integrally with at least one of the carrier sections, being suitable for fixing the sections in a folded state and to release the surface sections for transforming gradually or stepwise into an unfolded state. The holding mechanism is made of a material providing a dissolution behavior which dissolves in liquid environment faster than the remaining carrier structure.

In contrast to the elastic solid joint the carrier structure is made of stiffer material advantageously to ensure that the free accessible surface of the carrier structure remains unchanged and a defined amount of API can be released during the intracorporal retention period. Advantageously both the carrier structure as well as the solid joint is made of biodegradable and for bio-availability suited material which dissolves completely after a predetermined intracorporal retention time.

Alternatively at least a part of the carrier structure and/or part of the solid joint can be made of biocompatible but otherwise non completely dissolving material which has to be released extracorporally as naturally.

In a preferred embodiment the solid joint is made of smart material which enables to incorporate a technical function, for example to start the unfolding mechanism in a controlled manner or to activate a refolding process after a defined intracorporal retention time. Basically there are a multitude of possible smart materials for the use of building a solid joint by generative manufacturing method. Among known smart materials the following smart materials provide preferred functionality for realizing the solid joint: shape memory alloys, shape-memory polymers, electro- or magnetostrictive materials, magnetic shape memory alloys, smart inorganic polymers, pH-sensitive polymers, temperature-responsive polymers, photomechanical materials, dielectric elastomers, magnetocaloric materials.

A solid joint made of shape-memory material for example ensures an endurable maintenance of a predefined shape after unfolding the carrier structure.

Using smart materials which can be activated or triggered by an external influence for example by changing the intracorporal chemical environment by introducing a special material into the body for example by infusion, the process of unfolding and/or folding the carrier structure can be activated and/or repeated as many times as possible. Furthermore the process of unfolding or folding can be performed at least partially.

In the same way of altering chemical environment as a trigger mechanism which can be applied extracorporally many other physical parameters depending on the smart material of which the solid joint is made of can be chosen as a control parameter for activating the solid joint function as explained before. In case of electro- or magnetostrictive materials or magnetic shape memory alloys of which the at least one solid joint is made of, a controlled application of an external electrical, magnetical or electromagentical field acting within the body where the dosage form stays has an effect on the smart material and influences the function of the solid joint. In case of temperature-responsive polymers of which the at least one solid joint is made of, a controlled temperature change within the body can activate the solid joint function as well. In case of photomechanical materials of which the at least one solid joint is made of, a controlled application of electromagnetic radiation, for example X-ray, can activate the solid joint function as well.

Such kind of external trigger mechanism can enable to reduce shape and size of the carrier structure in its original state which is small enough to pass through natural intracorporal passages to be excreted surely and safely.

A preferred embodiment of a dosage form provides a carrier structure with a multitude of sections all linked together by solid joints. All solid joints may be of same material which is preferably smart material, so that activation of all solid joints at once can be triggered as described before.

In another embodiment of a dosage form the multitude of sections of the carrier structure is linked by solid joints. At least some of the solid joints are made of different materials, preferably different smart materials which are activated by different physical parameters. In this way one carrier structure can be folded or unfolded in some regions differently by changing different external parameters which leads to a high degree of activating the function of all solid joints in one carrier structure.

A further advantage of an externally controlled function of the solid joint concerns the possibility of influencing the amount of API delivery into the body. Controlling the folding mechanism by an external trigger the carrier structure can be transformed from a folded state in which the API delivery is minimized into a completely unfolded state in which the API delivery is maximized. The transformation between both states can be varied continuously or stepwise. For example, found that an API does not show the desired therapeutical effect or that an API deteriorate a biological intracorporal state than the triggerable solid joint can be activated to close respectively fold the carrier structure for the purpose of a quick extracorporal release.

In the event that just only the solid joint is fabricated by a generative manufacturing method the at least two sections of the carrier structure have to be provided in advance. In case of a two dimensional carrier structure the at least two sections can be separated from a carrier substrate like a biocompatible foil or film. Cutting is performed preferably on basis of a binary data set which is decisively for the shape, size and also functionality of the whole carrier structure. The at least two separate sections have to be integrated in a generative manufacturing process by positioning both sections close together so that the generative manufacturing method for producing the solid joint can be applied. In the process the solid joint will be produced in layers wherein the building material of the solid joint and the separate sections of the carrier structure form an integral material connection.

Advantageously the solid joint as well the carrier sections will be manufactured in way of a generative manufacturing method. In a first step a numerical model of the carrier structure which is defined by size, shape and kind of materials is generated to obtain a binary data set. Advantageously when generating the numerical model of the carrier structure the folding and unfolding mechanism has to be defined by using interactive developing tools for origami technique to create two or three dimensional structures in which folding bibliotheca or templates are implemented. By using these tools a folding and unfolding plan can be obtained in an economical way and a multitude of different configurations of foldable carrier structures can be obtained individually depending on therapeutic effect, nature of each person, etc. Further the defined and calculated folding pattern can determine the folding direction and order in which the folding and unfolding will occur. It is conceivable that the unfolding process can be carried out stepwise so that the carrier structure will have different special configurations in different intracorporal regions. Especially by using smart material which can be triggered from outside the folding action which is carried out by the at least one solid joint can be performed in a predetermined manner.

On the basis of the before described binary data set the whole dosage form comprising the carrier structure as well the at least one solid joint can be build in way of a generative manufacturing method in layers.

So at least one section of the carrier structure consists advantageously of at least two integrally joint layers one above the other of two different materials and/or two different API. Preferably polymer materials are used for deposition in layers serving as a matrix in which at least the one API is contained. When using generative manufacturing method for obtaining the carrier structure and the at least one solid joint it is advantageous that at least one section of the carrier structure provides a gradually or stepwise profile concerning at least one of the following features: concentrations API, solubility in liquid.

Of course the application of the novel dosage form is not limited to medications, rather it is advisable to extend the application to non-pharma products, like food, dieting and health care industry.

In the following the advantageous of the new dosage form will be summarized in note form:
- personalized dosage forms becomes possible
- reduce of the amount of excipients
- use of multi-API in one dosage form
- increase of size and surface of the dosage form in the body in order to extend the stay in a targeted area to allow for extended release
- complex carrier structures and automated folding are possible
- combination of thin sheet API in multi-layer setup bonded via laser welding
- dosage can be adjusted by choosing size of the dosage form
- release profile can be programmed and structured
- dosage will be created numerically and afterwards manufactured by generative manufacturing methods, preferably in a flatbed manner in order be minimized during the folding process.
- folding process is partially reversible in order to release the structure and API
- complex carrier structures are possible to cater to the personalized body condition
- folded scribe lines are enforced locally via laser by cutting off a film or foil and/or by 3D-printing
- automated folding by using shrink rate of elastomer, release can also be in a profile by dissolving the print material at different speeds than the sheet material
- carriers sections can be added or assembled from one to another to combine API in the same carrier structure
- application to non-pharma like food, dieting and healthcare industry
- Minimum of excipients needed

## Claims

1. Dosage form comprising a carrier structure with at least two sections adjoining each other integrally by a linearly solid joint, at least one section provides at least one active pharmaceutical ingredient (API) and/or pharmaceutically acceptable excipient and/or drug product formulation,
**wherein** at least the solid joint is produced by a generative manufacturing method.

2. Dosage form comprising a carrier structure with at least two sections adjoining each other integrally by a linearly solid joint, at least one section provides at least one active pharmaceutical ingredient (API) and/or pharmaceutically acceptable excipient and/or drug product formulation,
**wherein** the solid joint is produced by irradiation at least one surface of the carrier structure locally by a laser beam which transforms and/or removes material of the carrier structure partially, and/or
the solid joint is produced by introducing a surface cut at least at one surface of the carrier structure by a mechanical cutting tool which penetrates the carrier surface without complete separation.

3. Dosage form according to claim 1 or 2,
**wherein** the solid joint differs at least from the at least two adjoining sections in one of the following features: material, density, flexibility, geometry concerning thickness and/or shape, solubility in liquid, thermal, electrical, magnetical and/or electromagnetic absorption ability.

4. Dosage form according to one of the claims 1 to 3,
**wherein** the solid joint is made of a smart material comprising at least one of the following smart materials: shape-memory alloys, shape-memory polymers, magnetostrictive materials, magnetic shape memory alloys, smart inorganic polymers, pH-sensitive polymers, temperature-responsive polymers, photomechanical materials, dielectric elastomers, magnetocaloric materials.

5. Dosage form according to one of the claims 1 to 4,
wherein at least one section consists of at least two integrally joined layers one above the other of two different materials and/or two different active pharmaceutical ingredients (API) and/or pharmaceutically acceptable excipients and/or drug product formulations.

6. Dosage form according to claim 5,
wherein at least one of the two different materials is a smart material.

7. Dosage form according to one of the claims 1 to 5,
wherein the at least two sections adjoining each other integrally by the linearly solid joint are manufactured by the generative manufacturing method.

8. Dosage form according to one of the claims 1 and 3 to 5,
wherein the at least two sections are provided by cutting each surface section from a film or foil.

9. Dosage form according to one of the claims 1 and 3 to 7,
**wherein** the a generative manufacturing method is an additive manufacturing process.

10. Dosage form according to one of the claims 1 to 9,
**wherein** at least one sections of the carrier structure provides a gradually or stepwise profile concerning at least one of the following features: concentrations of active pharmaceutical ingredient (API) and/or pharmaceutically acceptable excipient and/or drug product formulation, solubility in liquid.

11. Dosage form according to one of the claims 1 to 10,
**wherein** the carrier provides a holding mechanism connected integrally with at least one of the sections, being suitable for fixing the sections in a folded state and to release the surface sections for transforming gradually or stepwise into an unfolded state.

12. Dosage form according to one of the claims 1 to 11,
**wherein** the solid joint is designed such that an unfold and a folding action is feasible.

13. Dosage form according to one of the claims 1 to 12,
wherein the carrier is a two dimensional carrier and the sections are surface sections which are joined by the solid joint.

14. Method for manufacturing a dosage form according to one of the claims 1 and 3 to 13,
**comprising** the steps of
- generating a numerical model of the carrier defining by size, shape and kind of materials to be used for building the sections adjoining each other integrally by a linearly solid joint and to obtain a binary data set, and
- building the dosage form on basis of the binary data set by a generative manufacturing method.

15. Method for manufacturing a dosage form according to one of the claims 1 to 13,
**comprising** the steps of
- generating a numerical model of the carrier defining by size, shape and kind of materials to obtain a binary data set,
- Cutting and providing at least two sections from a carrier substrate on basis of the binary data set and
- building the dosage form by generating the solid joint between the at least two sections on basis of the binary data set by a generative manufacturing method.

16. Method for manufacturing a dosage form according one of the claims 2 to 13, **comprising** the steps of
- generating a numerical model of a geometrical pattern of the solid joint adjoining the at least two sections of the carrier structure to obtain a binary data set,
- irradiating at least one surface of the carrier structure locally by a laser beam being deflected along the surface on basis of the binary data set for transforming and/or removing material locally of the carrier structure in resulting of the at least one solid joint and/or
- introducing at least one surface cut at least at one surface of the carrier structure by a mechanical cutting tool which penetrates the surface of the carrier structure without complete separation and which is carried relative to the carrier structure on basis of the binary data set.

17. Method according to one of the claims 15 or 16,
**wherein** for generating the numerical model data of a folding bibliotheca or templates are used.

18. Method according to one of the claims 15 to 17,
**wherein** applying a defined and calculated folding pattern determining the folding direction and order in which the folding and unfolding will occur.
